(19) Europäisches Patentamt
European Patent Office
Office européen des brevets

(11) Publication number: **0 192 059**
**B1**

(12) **EUROPEAN PATENT SPECIFICATION**

(45) Date of publication of patent specification: **20.09.89**

(51) Int. Cl.[4]: **C 07 C 11/02**, C 07 C 5/373, B 01 J 23/26

(21) Application number: **86100704.5**

(22) Date of filing: **20.01.86**

(54) **Dehydroisomerization of hydrocarbons.**

(30) Priority: **22.01.85 US 693494**

(43) Date of publication of application:
**27.08.86 Bulletin 86/35**

(45) Publication of the grant of the patent:
**20.09.89 Bulletin 89/38**

(84) Designated Contracting States:
**BE DE FR GB IT NL**

(56) References cited:
**EP-A-0 042 252**
**US-A-2 346 657**

(73) Proprietor: **AIR PRODUCTS AND CHEMICALS, INC.**
**Route no. 222**
**Trexlertown Pennsylvania 18087 (US)**

(72) Inventor: **Kirner, John Francis**
**404 Benner Road, Apt. 201**
**Allentown PA 18104 (US)**

(74) Representative: **Dipl.-Ing. Schwabe, Dr. Dr. Sandmair, Dr. Marx**
**Stuntzstrasse 16**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 192 059 B1

**Description**

Technical field

The present invention relates to catalytic dehydroisomerization of hydrocarbons and is more specifically concerned with method and catalyst for the direct production of iso-olefins from the corresponding normal paraffins.

Background of the invention

Among the increasing industrial demands for isobutylene is that for use in reaction with methanol to produce methyl-t-butyl ether (MTBE), valuable as a high octane blending component for automotive fuel. The increasing demand is straining the normal supply of isobutylene obtained from catalytic cracking and steam cracking processes. Of course, isobutylene can be readily obtained by dehydrogenation of isobutane by known processes, such as by the commercially available Catofin™ route (Gussow, S., et al., Oil and Gas J., Dec. 1980, pp 96—101) but the demand for isobutane as such as a high octane blending stock, has limited its supply and pushed up its market price because of the cost involved in isomerizing normal butane to meet the demand for isobutane.

In typical prior art processes a two step operation is employed, wherein normal butane is first isomerized under particular operating conditions and appropriate catalyst, and the thus obtained isobutane is subjected to dehydrogenation to obtain the desired isobutylene.

While the direct conversion of normal butane to isobutylene in a single step is obviously desirable from economic considerations, a primary deterrent to successful operation of this scheme has been the thermodynamic incompatibility of the isomerization and dehydrogenation steps as carried out in existing processes.

In the two step process for the conversion of n-butane to isobutylene, the initial isomerization of n-butane to isobutane would likely employ the typical commercially available first step using platinum catalyst on an acidic support, in the presence of added hydrogen at moderate total pressure and at temperatures above about 148°C (300°F). The resulting isobutane produced, separated from other reaction products, can be separately dehydrogenated by one of the known methods, such as the Catofin process for example, using a chrome-alumina catalyst at high temperatures (550—600°C) and at low pressure (sub-atmospheric).

Among recent patents disclosing conversion of hydrocarbons by multi-step operation, including dehydrogenation and isomerization, are:

U.S. 4,725,255; 4,324,937; 4,329,516, 4,341,911; and 4,367,357.

According to U.S. Patent 3,702,876 selective dehydrogenation of hydrocarbons can be achieved by pretreatment of a Pd catalyst with the desired olefin using a hollow tube palladium catalyst as a hydrogen permeable membrane. Only olefins which have the same skeleton as the charge hydrocarbon can be formed thereby.

While in certain reforming processes applied to various streams of mixed hydrocarbons a certain degree of dehydrogenation may take place accompanied by isomerization, none of these reforming processes have been commercially considered as a route for conversion of normal paraffin components in a $C_4$ or $C_5$ hydrocarbon stream to the corresponding iso-olefins.

Of interest from an academic viewpoint are the disclosure (1) by Nahm, J. J. West, et al., in *Proc. Int. Clean Air Cong.* 4th, 491 (1977), of the Photocatalytic conversion of hydrocarbons (1 ppm) adsorbed on airborne particulate containing trace metals (CA 88: 157695v); and that (2) by Matsuoka, S. et al., on the radiation sensitized thermal cracking of n-butane to isobutane and isobutylene (CA 83: 50623q).

US—A—2 346 657 describes a process for producing isobutene and n-butenes from normal butane, which comprises contacting such normal butane at a temperature in the range of 482 to 593°C under substantially atmospheric pressure with the catalyst mixture comprising certain refractory oxides and silicates having catalytic ability in the dehydrogenating reactions, and which are improved in this catalytic ability by the addition of catalysts in minor proportions, which comprise compounds and oxides of the elements of groups IVa, Va and VIa of the Periodic Table.

It has been recently proposed in published European patent application 42,252 (96 CA 180741f) to prepare isobutylene by dehydroisomerization of normal butane at 350—700°C over catalyst comprised of a Group IIIB metal or compound, specifically gallium oxide, deposited on a support having low acidity, such as $Al_2O_3$, $SiO_2$, or an aluminosilicate zeolite. In the given example the hydrocarbon feedstock containing n-butane was passed over the catalyst, comprising Ga zeolite L, at atmospheric pressure, at 550°C and at a contact time of 6 seconds. A conversion of 55 wt.% is reported to have been obtained at a yield of 2.5 wt.% aromatics and 28.9% butenes fraction comprising about 33% isobutylene (=about 9.5% isobutylene yield).

Among the objects of the present invention is the provision of an operative single step process for direct conversion of $C_4$ and/or $C_5$ paraffins to the corresponding iso-olefins, and to provide a novel catalyst promoting such dehydroisomerization.

Subject of the present invention is a process for the production of iso-olefines from normal paraffines of 4 to 5 carbon atoms according to Claim 1.

Preferred embodiments of this process are subject matter of Claims 2 to 5.

A further object of the present invention is a dehydroisomerization catalyst according to Claim 6, a preferred embodiment of which is subject of Claim 7.

Still a further subject is a method of preparing a dehydroisomerization catalyst according to the present invention, as claimed in Claim 8.

Summary of the invention

In accordance with the present invention normal butane, alone or in a mixed $C_4$ hydrocarbon-containing charge stream, is converted to isobutylene by contact of the $C_4$ charge with a catalyst comprising from 1 to 20% by weight of chromia ($Cr_2O_3$) and from 2 to 10% by weight of niobium pentoxide supported on a $ZrO_2$-carrier under operating conditions including temperature in the range of about 450—650°C, and at a $C_4$ pressure of from $10^3$ to $5\times10^4$ Pa (0.01 to about 0.5 atmospheres). The butane contact time is preferably 0.4 to 0.8 seconds.

The process is also applicable to the dehydro-isomerization of normal pentane alone or in a mixed hydrocarbon stream which may also comprise normal butane.

Any form of pressure-retaining catalytic reactor of the type employed in dehydrogenation of hydrocarbons may be employed in practice of the invention, including suitable means for fractional separation of the reaction products and provision for recyling of selected fractions.

Detailed description

In a laboratory program directed to investigation of activity of various catalysts tested for dehydrogenation of isobutane it was discovered that $Nb_2O_5$ supported on $ZrO_2$ added substantial isomerization activity to the intrinsic dehydrogenation activity of the zirconia support. As a result of this initial discovery further laboratory work was initiated which led to the development of the novel dehydroisomerization catalyst of the present invention, comprised essentially of $Cr_2O_3$ and $Nb_2O_5$ supported on a carrier having dehydrogenation activity, preferably on zirconia ($ZrO_2$).

Example A

In five minute onstream runs carried out for the dehydrogenation of 17% isobutane in a helium carrier gas at 600°C zirconia alone showed high dehydrogenation selectivity at comparatively low $iC_4$ conversion, while under the same operating conditions a run made using $Nb_2O_5$ supported on the zirconia carrier, showed significant isomerization activity but substantially less dehydrogenation activity. The results are reported in Table 1.

TABLE 1

| Run No. | Catalyst | $iC_4$ conversion wt.% | $iC_4$= selectivity wt.% | *$nC_4$= selectivity wt.% | $nC_4$ selectivity wt.% |
|---|---|---|---|---|---|
| 1 | $ZrO_2$ | 10 | 97 | <1 | <1 |
| 2 | $Nb_2O_5/ZrO_2$ | 30 | 45 | 22 | 1 |
| 3 | $Cr_2O_3/Nb_2O_5/ZrO_2$ | 66 | 63 | 7 | 0 |

* all isomers of n-butylene

Example 1

A catalyst composed of $Cr_2O_3/Nb_2O_5/ZrO_2$ (0.1/0.04/1 moles) was prepared and tested on the same $iC_4$ charge stock and under conditions of Example A above. The results are reported in Table 1.

The foregoing run (3) on the $Cr_2O_3/Nb_2O_5/ZrO_2$ catalyst indicated that it was an active catalyst for dehydrogenation of paraffins while retaining substantial olefin isomerization activity. Although the reported initial results indicated that the isomerization activity had somewhat decreased, it was recognized that the proportions of the components in the catalyst and the process conditions had not been optimized.

It will be noted from the results of Run 3 of Table 1, that none of the starting isobutylene was isomerized to n-butane.

The catalyst employed in Example 1 was prepared as follows:

(a) In the preparation of the $ZrO_2$ substrate, 600 grams of zirconyl nitrate (19.9 wt.% $ZrO_2$, 21.9% $HNO_3$) were diluted to 3 liters with distilled water. Precipitation was effected by slow addition of 250 ml of concentrated $NH_4OH$ with stirring during a 20 minute period. The product was let to set for 3 hours and filtered. The precipitate was washed 3 times with distilled water and dried at 120°C for 24 hours; after which the product was ground and sieved through a 20 mesh screen.

(b) 80.7 grams of niobium oxalate were added to an aqueous solution of oxalic acid comprised of 27 grams oxalic acid in 300 ml distilled water. After sitting over the weekend, the niobium oxalate was not completely dissolved. 25 ml of the supernatant liquid (considered to be a saturated solution of niobium

oxalate in 1 molar aqueous oxalic acid) was added to 15 grams of the screened $ZrO_2$ substrate from (a) above. After 30 minute contact of the substrate with the niobium oxalate solution with occasional stirring, the impregnated solid was filtered off and dried on a watchglass at 120°C for 24 hours. It was then heat treated in a muffle furnace at 650°C for 2 hours, obtaining a product comprised of $Nb_2O_5$ incorporated in $ZrO_2$ substrate.

(c) 15 grams of the $Nb_2O_5/ZrO_2$ product from (b) above was added to 25 ml of a solution containing 60 grams of $CrO_3$ dissolved in 100 ml $H_2O$. After sitting for 30 minutes with occasional stirring, the material was filtered and dried on a watchglass at 120°C for 15 hours; then heat treated in a muffle furnace at 650°C for 2 hours and 20 minutes. The obtained $Nb_2O_5/Cr_2O_3/ZrO_2$ product was used in the form of a 40×60 mesh powder; the surface area is about 35 $m^2/g$.

While not being bound to any particular theory as to the reaction mechanism involved it is reasonable to assume from the observed experimental results that the dehydroisomerization of normal butane would follow the mechanistic route indicated in equation (I) below:

$$\text{(I)}\quad nC_4 \rightarrow {}^*nC_4{=} \leftrightarrow iC_4{=}$$

($*nC_4$=mixture of normal $C_4$ olefins)

The maximum selectivity to isobutylene is determined by the equilibria involving the various $C_4$ mono-olefin isomers. Table 2 below shows the equilibria among these isomers (in mole fractions) as a function of temperature.

TABLE 2

| Olefin | 450°C | 550°C | 650°C |
|---|---|---|---|
| 1-butene | 0.12 | 0.15 | 0.18 |
| 2-butene (cis) | 0.16 | 0.17 | 0.17 |
| 2-butene (trans) | 0.24 | 0.25 | 0.25 |
| isobutylene | 0.48 | 0.44 | 0.41 |

In considering the equilibria among the various $C_4$ mono-olefin isomers pressure was ruled out as a process variable, since neither the total pressure nor the partial pressure of hydrogen has a significant effect on the equilibria obtaining and thus pressure could not be used as a means of optimizing isobutylene selectivity. Maximum obtainable conversion at thermodynamic equilibrium, however, is a function of pressure and, therefore, governs total yield of iso-olefins.

From the equilibrium data in Table 2, it will be seen that the formation of isobutylene is favored as the temperature is lowered. Temperatures below about 450°C, however, would not be sufficient to further the reaction at a desired reasonable rate. Accordingly, selectivity to isobutylene formation for an optimized process appears to be at about 45%, obtainable at temperatures in the range of 450—550°C. To maximize production of isobutylene, about 55% of the mono-olefin products obtained can be recycled to the dehydroisomerization reaction. Moreover, in establishing the process conditions to be utilized for the desired product yield, consideration needs to be given to avoiding the kinetic routes favoring isomerization of butane to isobutane, formation of cracked products, high yields of coke.

The effect of pressure on $C_4$ dehydrogenation equilibria at various temperatures is shown in Table 3.

TABLE 3
$C_4$ Dehydrogenation equilibria
(mole fractions)

| Temperature (°C) | 650 | 550 | 450 |
|---|---|---|---|
| Pressure in Pa (atm) | $10^4$ (0.1) | $10^4$ (0.1) | $10^4$ (0.1) |
| $H_2$ | 0.607 | 0.516 | 0.375 |
| n-$C_4$ | 0.003 | 0.035 | 0.257 |
| 1-$C_4$= | 0.031 | 0.056 | 0.042 |
| 2C-$C_4$= | 0.030 | 0.063 | 0.058 |
| 2T-$C_4$= | 0.043 | 0.093 | 0.088 |
| 1,3-BD | 0.217 | 0.070 | 0.066 |
| i$C_4$= | 0.071 | 0.166 | 0.174 |
| Pressure in Pa (atm) | $3\times10^4$ (0.3) | $3\times10^4$ (0.3) | $3\times10^4$ (0.3) |
| $H_2$ | 0.560 | 0.464 | 0.280 |
| n-$C_4$ | 0.014 | 0.101 | 0.441 |
| 1-$C_4$= | 0.052 | 0.060 | 0.032 |
| 2C-$C_4$= | 0.050 | 0.068 | 0.044 |
| 2T-$C_4$= | 0.072 | 0.100 | 0.067 |
| 1,3-BD | 0.132 | 0.028 | 0.022 |
| i$C_4$= | 0.120 | 0.179 | 0.133 |

In the single step conversion of n-butane to isobutylene the following operation conditions may be employed.

| | Range | Preferred |
|---|---|---|
| Temperature, °C | 450—650 | 550—600 |
| $C_4$ Pressure, Pa (atm.) | $10^3$–$5\times10^4$ (0.01—0.5) | $10^3$–$2\times10^4$ (0.1—0.2) |
| $H_2$/feed mole ratio | 0—10 | 0—4 |
| Contact time, sec. | 0.1—1.0 | 0.4—0.8 |
| *Space rate, v/hr/V | 120—560 | 240—480 |

*at STPn-$C_4$

The catalyst to be employed comprises $Cr_2O_3$ and $Nb_2O_5$ supported on a carrier having dehydrogenation activity, which comprises in calcined form essentially 1.90 to 8.5% by weight of $Cr_2O_3$ and 2.86 to 5.98% by weight of $Nb_2O_5$ (0.02 to 0.10 parts $Cr_2O_3$ and 0.03 to 0.07 parts $Nb_2O_5$) on a support consisting essentially of $ZrO_2$; preferably 5.83% by weight of $Cr_2O_3$ and 4.46% by weight of $Nb_2O_5$ (0.07 parts $Cr_2O_3$ and 0.05 parts $Nb_2O_5$) on said support.

The reaction products are separated to recover isobutylene; products boiling above isobutylene being recycled to join the fresh butane feed.

In the dehydroisomerization of normal pentane the reaction conditions may be essentially the same as above set out for normal butane.

The catalyst may be prepared by impregnating the zirconia carrier with an aqueous solution containing niobium, drying and/or calcining, and impregnating the resulting solid with an aqueous solution containing chromium, followed by drying and calcining.

## Claims

1. A process for the production of iso-olefins from normal paraffins of 4 to 5 carbon atoms which comprises contacting the normal paraffin change with a catalyst comprising oxides of the elements of groups IVa, Va and VIa of the Periodic Table, supported on a carrier having dehydrogenation activity, under operating conditions including temperature in the range of 450—650°C, a gas hourly space velocity from 120 to 560 volumes of normal paraffin per volume of catalyst per hour, and at a normal paraffin pressure of from 0.01 to 0.5 atmospheres, characterized in that it comprises as the catalyst from 1 to 20% by weight of $Cr_2O_3$ and from 2 to 10% by weight $Nb_2O_5$ supported on a carrier which consists of $ZrO_2$.

2. The process as defined in Claim 1, wherein said catalyst comprises in calcined form 1.90 to 8.5% by weight of $Cr_2O_3$ and 2.86 to 5.98% by weight of $Nb_2O_5$.

3. The process as defined in Claims 1 or 2, wherein said contacting is carried out at temperature in the range of 550—600°C, paraffin pressure in the range of 0.1—0.2 atmospheres and at a gas velocity of 240—480 volumes normal paraffin at standard temperature and pressure per volume catalyst per hour.

4. The process as defined in any of Claims 1 to 3, wherein the normal paraffin charge comprises n-butane.

5. The process as defined in any of Claims 1 to 3, wherein the normal paraffin charge comprises n-pentane.

6. Dehydroisomerization catalyst comprising 1 to 20% by weight of $Cr_2O_3$ and 2 to 10% by weight of $Nb_2O_5$ on a carrier which consists of $ZrO_2$.

7. Catalyst according to Claim 6, comprising 1.90 to 8.5% by weight of $Cr_2O_3$ and 2.86 to 5.98% by weight of $Nb_2O_5$.

8. The method of preparing a dehydroisomerization catalyst according to Claim 6 or 7, which comprises impregnating a substrate carrier comprising $ZrO_2$ with an aqueous solution of a soluble niobium compound, drying and/or calcining, and impregnating the resulting solid with an aqueous solution of a soluble chromium compound, followed by drying and calcining.

## Patentansprüche

1. Verfahren zur Herstellung von Iso-Olefinen aus normalen Paraffinen mit 4 bis 5 Kohlenstoffatomen, bei dem man die normale Paraffinbeschickung mit einem Katalysator, der Oxide der Elemente der Gruppen IVa, Va und VIa des Periodischen Systems enthält, von einem Träger mit Dehydrierungsaktivität getragen wird, unter Betriebsbedingungen einschließlich einer Temperatur im Bereich von 450 bis 650°C, einer Gasstromgeschwindigkeit von 120 bis 560 Volumen Normalparaffin pro Volumen Katalysator pro Stunde und bei einem Normalparaffindruck von 0,01 bis 0,5 Atmosphären in Kontakt bringt, dadurch gekennzeichnet, daß als Katalysator 1 bis 20 Gew.-% $Cr_2O_3$ und 2 bis 10 Gew.-% $Nb_2O_5$ auf einem Träger, der aus $ZrO_2$ besteht, verwendet wird.

2. Verfahren nach Anspruch 1, bei dem der Katalysator in calcinierter Form 1,90 bis 8,5 Gew.-% $Cr_2O_3$ und 2,86 bis 5,98 Gew.-% $Nb_2O_5$ enthält.

3. Verfahren nach Anspruch 1 oder 2, bei dem das Inkontaktbringen bei einer Temperatur im Bereich von 550 bis 600°C, einem Paraffindruck im Bereich von 0,1 bis 0,2 Atmosphären und einer Gasgeschwindigkeit von 240 bis 480 Volumen Normalparaffin bei Standardtemperatur und Druck pro Volumen Katalysator pro Stunde durchgeführt wird.

4. Verfahren nach einem der Ansprüche 1 bis 3, bei dem die Normalparaffinbeschickung n-Butan enthält.

5. Verfahren nach einem der Ansprüche 1 bis 3, bei dem die Normalparaffinbeschickung n-Pentan enthält.

6. Dehydroisomerisierungskatalysator, enthaltend 1 bis 20 Gew.-% $Cr_2O_3$ und 2 bis 10 Gew.-% $Nb_2O_5$ auf einem Träger, der aus $ZrO_2$ besteht.

7. Katalysator nach Anspruch 6, enthaltend 1,90 bis 8,5 Gew.-% $Cr_2O_3$ und 2,86 bis 5,98 Gew.-% $Nb_2O_5$.

8. Verfahren zur Herstellung eines Dehydroisomerisierungskatalysators nach Anspruch 6 oder 7, bei dem man einen Schichtträger, der $ZrO_2$ enthält, mit einer wäßrigen Lösung aus einer löslichen Niobverbinung imprägniert, trocknet und/oder calciniert und den erhaltenen Feststoff mit einer wäßrigen Lösung aus einer löslichen Chromverbindung imprägniert, gefolgt von einer Trocknung und Calcinierung.

## Revendications

1. Procédé de production d'iso-oléfines à partir de paraffines normales de 4 à 5 atomes de carbone qui comprend la mise en contact de la charge de paraffine normale avec un catalyseur comprenant des oxydes des éléments des groupes IVa, Va, et VIa du Tableau Périodique, fixés sur un porteur ayant une activité de déshydrogénation, sous des conditions opératoires incluant une température comprise dans la gamme de

450—650°C, une vitesse spatiale horaire du gaz de 120 à 560 volumes de paraffine normale par volume de catalyseur par heure, et à une pression en paraffine normale de 0,01 à 0,5 atmosphère, caractérisé en ce qu'il comprend comme catalyseur de 1 à 20% en poids de $Cr_2O_3$ et de 2 à 10% en poids de $Nb_2O_5$ fixés sur un porteur consistant en $ZrO_2$.

2. Procédé selon la revendication 1, dans lequel le catalyseur comprend sous forme calcinée 1,90 à 8,5% en poids de $Cr_2O_3$ et 2,86 à 5,98% en poids de $Nb_2O_5$.

3. Procédé selon la revendication 1 ou 2, dans lequel le contact est effectué à une température comprise dans la gamme de 550—600°C, à une pression de la paraffine dans la gamme de 0,1—0,2 atmosphère et à une vitesse du gaz de 240—480 volumes de paraffine normale à température et pression standards par volume de catalyseur par heure.

4. Procédé selon n'importe laquelle des revendications 1 à 3, dans lequel la charge de paraffine normale comprend du n-butane.

5. Procédé selon n'importe laquelle des revendications 1 à 3, dans lequel la charge de paraffine normale comprend du n-pentane.

6. Catalyseur de déshydroisomérisation comprenant 1 à 20% en poids de $Cr_2O_3$ et 2 à 10% en poids de $Nb_2O_5$ sur un porteur consistant en $ZrO_2$.

7. Catalyseur selon la revendication 6, comprenant 1,90 à 8,5% en poids de $Cr_2O_3$ et 2,86 à 5,98% en poids de $Nb_2O_5$.

8. Procédé de préparation d'un catalyseur de déshydroisomérisation selon la revendication 6 ou 7, qui comprend l'imprégnation d'un porteur substrat comprenant $ZrO_2$ avec une solution aqueuse d'un composé de niobium soluble, puis un séchage et/ou une calcination, et l'imprégnation du solide résultant avec une solution aqueuse d'un composé de chrome soluble, suivie d'un séchage et d'une calcination.